(19) **Europäisches Patentamt / European Patent Office / Office européen des brevets**

(11) **EP 4 371 475 B1**

(12) **EUROPEAN PATENT SPECIFICATION**

(45) Date of publication and mention of the grant of the patent:
**19.11.2025   Bulletin 2025/47**

(21) Application number: **23150370.7**

(22) Date of filing: **04.01.2023**

(51) International Patent Classification (IPC):
*A61B 5/021* (2006.01)   *A61B 5/0215* (2006.01)
*A61B 5/318* (2021.01)   *A61B 5/00* (2006.01)
*A61B 5/107* (2006.01)   *A61B 8/08* (2006.01)
*A61B 8/00* (2006.01)

(52) Cooperative Patent Classification (CPC):
**A61B 8/0841; A61B 5/021; A61B 5/0215;**
**A61B 5/1073; A61B 5/1076; A61B 5/318;**
**A61B 5/6869; A61B 8/0883; A61B 8/4477;**
**A61B 8/5223;** A61B 5/6823; A61B 8/4209;
A61B 8/4488; A61B 8/4494

(54) **WIRELESS MEASUREMENT OF INTRABODY DIMENSIONS FOR PATIENT MONITORING AND DIAGNOSIS**

DRAHTLOSE MESSUNG VON INTRAKORPORALEN DIMENSIONEN ZUR PATIENTENÜBERWACHUNG UND -DIAGNOSE

MESURE SANS FIL DE DIMENSIONS INTRACORPORELLES POUR LA SURVEILLANCE ET LE DIAGNOSTIC DE PATIENTS

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority:   **15.11.2022   US 202263425641 P**

(43) Date of publication of application:
**22.05.2024   Bulletin 2024/21**

(73) Proprietor: **Tau Cardia Ltd**
**Yokneam Illit 2066733 (IL)**

(72) Inventor: **REUVEN M, Lewinsky**
**3600500 Alonei Aba (IL)**

(74) Representative: **KIPA AB**
**Drottninggatan 11**
**252 21 Helsingborg (SE)**

(56) References cited:
US-A- 5 201 715          US-A1- 2018 177 486
US-A1- 2022 079 550      US-A1- 2022 265 157

**Description**

Field of the Invention

The present invention relates generally to the field of diagnostic medicine, in particular to intrabody measurements using implantable medical devices such as wireless sensors, enabling patient monitoring and diagnosis, as well as related systems, devices and software products.

Background of the Invention

Remote monitoring of patients with heart disease, especially those suffering from Congestive Heart Failure (CHF) is very challenging. There are several methods for observation of such patients including monitoring of symptoms, weight gain, changes in the ECG, thoracic impedance, and other parameters. These, however, are of limited value due to suboptimal performance (for example in terms of sensitivity, specificity, and rapidity).

Currently, there is no optimal device/method to detect cardiac deterioration at its initial phase, when such detection could prevent further deterioration, hospitalization and even death.

This section is intended to introduce the reader to various aspects of art that may be related to various aspects of the present disclosure, which are described and/or claimed below. This discussion is believed to be helpful in providing the reader with background information to facilitate a better understanding of the various aspects of the present disclosure. Accordingly, it should be understood that these statements are to be read in this light, and not as admissions of prior art.

Recently, several devices have been introduced into clinical practice which enable the measurement of Left Atrial Pressure (LAP), an increase of which is considered to be a predictor of deterioration of CHF. This is achieved by deploying a pressure sensor either in the Pulmonary Artery (indirect measurement, or in the Left Atrium which measures and wirelessly transmits the results. These devices show some advantages over past methods but their performance is still suboptimal, relying on the measurement of a single parameter which is often not indicative of a specific person's condition.

The gold standard in investigational cardiology for the assessment of myocardial function is the measurement of the volume of cardiac chambers throughout the cardiac cycle (systole and diastole) and correlating them with simultaneous pressure measurements yielding Pressure-Volume Loops (PVL) where each cardiac cycle is represented by a single loop (Sagawa, The End-systolic Pressure-Volume Relation of the Ventricle: Definition, Modifications, and Clinical Use, Circulation 63:6 pp. 1223-1227. 1981). The analysis of these loops, either single or a sequential series of several cardiac cycles provides valuable information regarding important parameters of cardiac function such as myocardial contractility during systole (inotropy), myocardial relaxation during diastole (lusitropy), preload and afterload. This is currently only possible in experimental models or by using a conductance catheter to yield continuous volume measurements, an invasive method not applicable for routine medical practice (Baan et al, Continuous measurement of left ventricular volume in animals and humans by conductance catheter, Circulation 70:5, pp 812-823 1984; and Burkhoff, Pressure-Volume Loops In Clinical Research, J. American College of Cardiology 62:13 pp 1173-1176 2013). Cardiac chamber volumes can also be assessed by different modalities such as echocardiography, CT or MRI. These however, are only available in hospitals or clinics, require professional staff and are, therefore, not suitable for remote ambulatory monitoring.

Echocardiographic measurements of cardiac chamber volumes are also known to be prone to inter-observer variability and even serial measurements taken by the same operator may yield varying results since it is difficult to assure exact repeatability of the plane or location in which they are taken.

Recently, methods for automatic processing of echocardiographic images are being used to measure cardiac chamber dimensions. These include among others the speckle tracking technology or the enhancement of analysis by applying artificial intelligence (AI) algorithms (US 10078893 "Automatic Left Ventricular Function Evaluation" to Guterman et al.). These methods, however, still require the acquisition of high-quality echocardiographic images, a task which requires trained physicians or technicians thus making them unsuitable for home monitoring of cardiac patients. Furthermore, a large proportion of heart failure patients, have a preserved ejection fraction (EF). These cases which commonly result from myocardial diastolic dysfunction are termed 'heart failure with preserved ejection fraction' (HFpEF) and as such, their condition is often not accurately assessed by relying on the detection of cardiac chamber contours and measurement of volumes alone without correlating with simultaneous pressure measurements. The current practice for the diagnosis of HFpEF mostly relies on measurements of flow across the mitral valve and mitral annular motion velocity performed by Doppler echocardiography. These, however, are subject to operator skills, suffer from high inter-observer variability and are influenced by various other parameters. A consistent, reliable method for the diagnosis of HFpEF is not yet available for routine medical use.

Currently, there is no device or method suitable for reliable, user-friendly acquisition of cardiac pressure-volume loops, considered to be the gold standard for the assessment of ventricular function, in routine cardiology practice. The ability to introduce measurement and monitoring of parameters derived from cardiac pressure-volume loops can significantly contribute to the management of heart failure and other cardiac disease, improve patient's quality of life and reduce healthcare costs. US2022265157 A1 from Charthad Jayant et al (2022-08-25) discloses a system that uses active position ultrasound sensors within the heart for determining pressure-volume loops and D2, US2018177486 A1 from Gifford, III, Hanson et al (2018-06-28) discloses a system that uses passive ultrasound sensors which can be applied at the heart.

Summary of the Invention

The invention is defined in the appended claims.

The disclosure includes methods, devices/apparatuses/systems, a computer-readable media, and software products at least suitable or configured for continuous or intermittent wireless monitoring of bodily parameters such as the volume of cardiac chambers. As such, it can provide important information regarding myocardial as well as heart valve function. When combined with pressure measurements, optimal remote monitoring and/or diagnosis of cardiac patients can be achieved, in particular non-invasively. Thus, the invention provides a wireless sensing solution to monitoring of cardiac chamber volumes as well as PVL, effectively providing the most important parameters of cardiac function, without the need to acquire and analyze echocardiographic images by trained medical professionals. The method can be customized according to each patient's specific medical condition.

The disclosure is designed as a foolproof device to be operated by the patient, a family member or any other non-professional, which enables remote monitoring of CHF patients, by wireless measurement of the volume of and pressure in cardiac chambers to create Pressure-Volume Loops which provide the best estimate for cardiac function, in a continuous or intermittent manner.

The main components of a primary example include:

- A set of wireless location tracking sensors (these being passive reflectors adapted for use with ultrasound signals). These are implanted in the body at locations that are prone to relative movement, for instance at various points on the wall of a cardiac chamber. Once implanted, the location of the previously implanted sensors can be continuously tracked and the distance between them calculated. This enables (for instance) the determination of the volume of the cardiac chamber (i.e., the left ventricle).
- Means for sensing and transmitting the pressure within one or more cardiac chamber(s) (including cardiac atria and ventricles), again ideally using wireless means.
- An external device adapted to track the wireless location sensors previously implanted, to determine their relative and/or absolute positions with respect to each other and/or the external device, as well as to take readings from the pressure sensor(s) and generate a series of PVLs of the cardiac chamber for further analysis. The external device will generally comprise one or more ultrasound transducers, possibly in the form of linear or matrix arrays.

The location tracking sensors are passive reflectors for use with ultrasound signals.

The pressure sensing may be accomplished for instance by means of further sensors, either as stand-alone sensors or alternatively integrated with some of the volume sensors of the disclosure.

The disclosure is primarily intended for continuous or intermittent monitoring with emphasis on outpatient environment. This is useful amongst others for patients suffering from congestive heart failure (CHF), valvular heart disease, intracardiac shunts, etc.

Placement of the position sensors assures that serial measurements over time are always made at the exact same location or plane which assures reliability and repeatability of measurements. This will enable the device to compare the results of each examination to those of previous examinations thus providing the ability to perform reliable trend analysis where each patient is his own control and not just determine if the results fit into commonly accepted population-based normal values. As such, the current disclosure may also be integrated into existing or future echocardiography devices used in clinics and hospitals to improve the diagnostic accuracy of routine echocardiographic examinations.

In one aspect of the disclosure, a system for wireless cardiac diagnostics is described. The system may include:

a. a set of wireless position sensors configured to be deployed in a cardiac chamber;
b. pressure sensing means adapted for sensing the pressure within the cardiac chamber;
c. an external device adapted to:

    i. determine the displacements between the wireless position sensors, when deployed, to provide displacement data;
    ii. determine the pressure from the pressure sensing means to provide pressure data,
    iii. to determine at least one pressure-volume loop (PVL) of a pressure and volume of the cardiac chamber based on the displacement data and pressure data;

iv. and determine preferably instantaneous measures of cardiac health based on the pressure-volume loop(s) (PVL),

whereby the system is configured to determine the cardiac health wirelessly, non-invasively, continuously, and without acquisition or analysis of high-quality echocardiographic images, the cardiac health preferably including existence and severity of heart failure, especially in a patient suffering from diastolic dysfunction or heart failure with preserved ejection fraction (HFpEF), but also regarding function or dysfunction of one or more cardiac valves on the left side of the heart and/or the right side of the heart, and/or wherein the measurements can be modulated by external manipulations of a body of the patient in order to produce a series of pressure-volume loops displaced relative to each other, for instance for calculation of end-systolic pressure-volume relationship (ESPVR), end-diastolic pressure-volume relationship (EDPVR) and other parameters of myocardial function related to the cardiac health.

In some examples of the disclosure, the wireless position sensors may be solid or hollow forms selected from the group consisting of: sphere, coil, cylinder, polyhedron, corner cube, ellipsoid, ring.

The wireless position sensors are passive ultrasound reflectors having high ultrasound reflectivity, and wherein the external device uses ultrasound transmitters and receivers to cause reflections from the wireless position sensors.

In some examples of the disclosure, the external device may be configured to use true range multilateration or triangulation to determine the displacement of the wireless position sensors.

In some examples of the disclosure, wherein the external device may be configured to extract and process raw spatial data from ultrasound transducer(s) to determine the displacement of the wireless position sensors.

In some examples of the disclosure, the pressure sensing means may be wireless.

In some examples of the disclosure, the pressure sensing means may be incorporated into one or more of the wireless position sensors.

In a further aspect of the disclosure, a method of cardiac health determination including noninvasive determination of cardiac pressure-volume loops is described. The method includes

a. continuously determining the displacement(s) between two or more position sensors previously affixed in or at a cardiac chamber, thus providing displacement data;
b. continuously determining the pressure in the cardiac chamber, preferably by a pressure sensor implanted in the cardiac chamber or a non-invasive cardiac pressure sensor or method, thus providing pressure data;
c. forming at least one pressure-volume loop from the displacement and pressure measurements' data;
d. calculating parameters of the at least one pressure-volume loop adapted to indicate cardiac health;

whereby cardiac health is determined wirelessly, non-invasively, continuously, and without the need to acquire or analyze high-quality echocardiographic images the cardiac health preferably including existence and severity of heart failure, especially in a patient suffering from diastolic dysfunction or heart failure with preserved ejection fraction (HFpEF),
and such measurement information is for instance derived regarding function or dysfunction of one or more cardiac valves on the left side of the heart and/or the right side of the heart, and/or wherein the measurements can be modulated by external manipulations of a body of the patient in order to produce a series of pressure-volume loops displaced relative to each other, for instance for calculation of end-systolic pressure-volume relationship (ESPVR), end-diastolic pressure-volume relationship (EDPVR) and other parameters of myocardial function related to the cardiac health.

In some examples of the disclosure, the wireless position sensors may be solid or hollow forms selected from the group consisting of: sphere, coil, cylinder, polyhedron, corner cube, ellipsoid, ring.

In some examples of the disclosure, the wireless position sensors may be passive ultrasound reflectors having high ultrasound reflectivity, and wherein the external device uses ultrasound transmitters and receivers to cause reflections from the wireless position sensors.

In some examples of the disclosure, the external device may use true range multilateration or triangulation to determine the displacement of the wireless position sensors.

In some examples of the disclosure, the external device may extract and process raw spatial data from the ultrasound transducer(s) to determine the displacement of the wireless position sensors.

In some examples of the disclosure, the pressure sensing means may be wireless.

In some examples of the disclosure, wherein the pressure sensing means may be incorporated into one or more of the wireless position sensors.

In another aspect of the disclosure, a system comprising an implantable medical device for monitoring variable body geometry is described. The system includes:

a. one or more wireless position sensors configured to be deployed at a set of bodily positions;

b. an external device adapted to:

i. determine the displacements between the wireless position sensors, when deployed, to provide displacement data;

ii. and determine instantaneous measures of cardiac health based on a pressure-volume loop;

whereby variable body geometry is preferably monitored continuously, noninvasively, and without the need to acquire or analyze high-quality echocardiographic images.

In some examples of the disclosure, the implantable medical device may further include at least one pressure sensor preferably deployed in the same cardiac chamber in which the position sensors are deployed, wherein the external device may further include means for determining the pressure from the pressure sensors.

In some examples of the disclosure, the wireless position sensors of the implantable medical device may preferably be placed within a cardiac chamber, such that the left cardiac chamber volume may be monitored and pressure-volume loops may be determined.

In yet another aspect of the disclosure, a software for cardiac health determination, including executable instructions for a processing unit of a device and performing the method described herein, is described. The cardiac health preferably including existence and severity of heart failure, including those suffering from diastolic dysfunction or heart failure with preserved ejection fraction (HFpEF).

i. In yet a further aspect of the disclosure, an external device for wireless cardiac diagnostics is described, the external device is adapted to: determine displacement data for displacements between wireless position sensors of a set of wireless position sensors deployed in or at a cardiac chamber;

ii. determine pressure data for pressure from a pressure sensing means adapted for sensing the pressure within the cardiac chamber;

iii. to determine a pressure-volume loop of a pressure and volume of the cardiac chamber based on the displacement data and pressure data;

iv. and determine instantaneous measures of cardiac health based on the pressure-volume loop;

whereby the device is configured to determine the cardiac health wirelessly, non-invasively, continuously, and without acquisition or analysis of high quality echocardiographic images the cardiac health preferably including existence and severity of heart failure, especially in a patient suffering from diastolic dysfunction or heart failure with preserved ejection fraction (HFpEF) and such measurement information is for instance derived regarding function or dysfunction of one or more cardiac valves on the left side of the heart and/or the right side of the heart, and/or wherein the measurements are modulated by external manipulations of a body of the patient in order to produce a series of pressure-volume loops displaced relative to each other, for instance for calculation of end-systolic pressure-volume relationship (ESPVR), end-diastolic pressure-volume relationship (EDPVR) and other parameters of myocardial function related to the cardiac health.

The foregoing examples have been described and illustrated in conjunction with systems and methods thereof, which are meant to be merely illustrative, and not limiting.

Brief Description of the Drawings

Specific embodiments of the invention will now be described with reference to the accompanying drawings. In the drawings, like numbers refer to like elements.

Embodiments and features of the present invention are described herein in conjunction with the following drawings:

Fig. 1 shows a block diagram of system components.

Fig. 2 shows a flowchart of one embodiment of system operation.

Fig. 3 shows a schematic of sensors in place in the heart.

Figs. 4A, B, C and D show several possible embodiments of the external unit of device.

Fig. 5 shows a typical pressure-volume loop (PVL) of the left ventricle.

Fig. 6 shows a position sensor which is comprised of several layers

Detailed Description of Preferred Embodiments

The present invention will be understood from the following detailed description of preferred embodiments, which are meant to be descriptive yet not limiting. For the sake of brevity, some well-known features, methods, systems, procedures, components, circuits, and so on, are not described in detail.

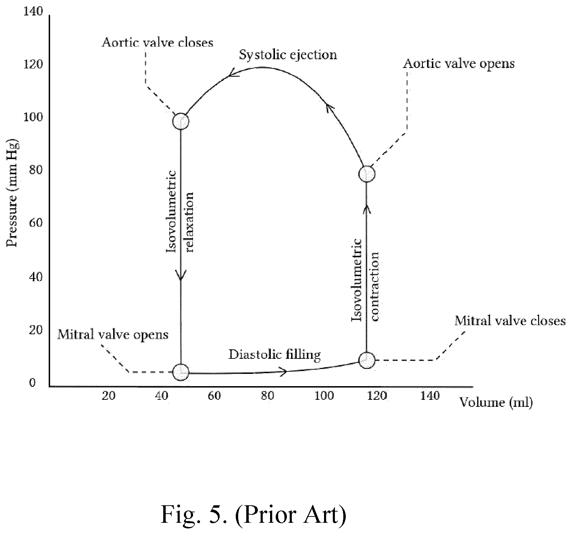

The 'gold standard' for assessing myocardial function and other critical parameters of cardiac function is the pressure-volume relationship for the left ventricle, this relationship being reflected in the 'PVL' or pressure-volume loop for left ventricle. For purposes of determining ventricular volume, either invasive means such as catheters are used, or ultrasound imaging and associated image processing have hitherto been employed. Invasive means have obvious drawbacks, while ultrasound with volume determination requires the acquisition and analysis of high-quality images by experts in Echocardiography, and even these are prone to interobserver variability. A representative PVL is shown in Fig. 5.

The current disclosure describes a method for continuous or intermittent wireless monitoring of the volume of cardiac chambers, with the ability to combine these with pressure measurements for optimal remote monitoring of cardiac patients, without the need to acquire or analyze high quality ultrasound images.

Monitoring of various bodily areas has been introduced for example in US 6498944 "Intrabody Measurement" to Ben Haim, which discloses a catheter-based method of measuring the size of a body area using a set of position sensors mounted on the tips of one or more catheters, and calculation of distances between them. The position sensors comprise miniature coils which reflect electromagnetic waves, which are produced and detected by suitable apparatus outside the body.

Similarly, US10918858 "Cardiac volume sensing via an implantable medical device in support of cardiac resynchronization therapy" discloses an implantable medical device having two electrodes and coupled to them, configured to identify a measure of impedance between the two electrodes.

Due to the obvious drawbacks of either using an invasive means such as a catheter or an active electrical measurement, it is an object of the current disclosure to introduce a method that avoids both the use of catheters and active impedance measurement for determination of time-varying bodily volumes. The main components of primary examples of the disclosure include one or more of:

- A set of wireless sensors deployed in a cardiac chamber. These are passive devices adapted to have high ultrasound reflectivity. Using true range multilateration, triangulation or similar means, or the spatial information which can be derived from the ultrasound transducer per its specific design, the location of the sensors can be continuously tracked and the distance between them calculated, to enable determination of the volume of the cardiac chamber (i.e., the left ventricle). The wireless sensors are passive reflectors which can be tracked by use of ultrasound signals.
- Means for sensing the pressure within one or more cardiac chamber(s).
- An external device adapted to track the wireless sensors, to determine their relative and/or absolute positions with respect to each other and/or the external device. The external device will generally comprise one or more ultrasound transducers, possibly in the form of linear arrays, phased arrays or matrix arrays.

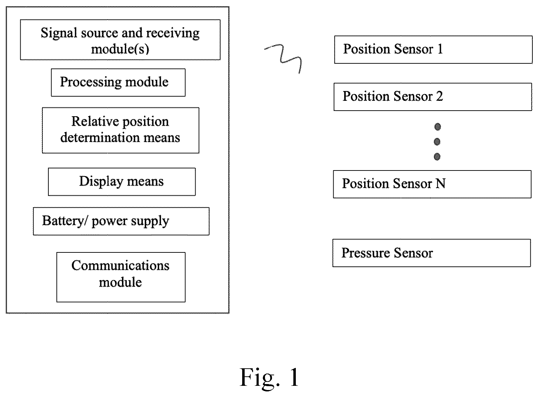

A block diagram of one embodiment of system components of a wearable external unit is shown in Fig. 1. On the left an external unit having a signal source and receiving module, ultrasound transducer elements, communication means, processing module, relative position determination means, display means and a power supply to power all these components is shown. The implanted sensors of the system are shown on the right, including two or more position sensors and at least one pressure sensor.

In another embodiment, the external unit of the disclosure, as a whole or part of it may also be integrated into existing or future echocardiography devices used in clinics and hospitals to improve the diagnostic accuracy of routine echocardiographic examinations.

The communication means of the external unit may include modules intended for communicating with other components of the system as well as with other devices such as PCs, smartphones, etc.

The display means of the external device will enable operation of the device as well as provide user feedback regarding signal quality, analysis of results, etc.

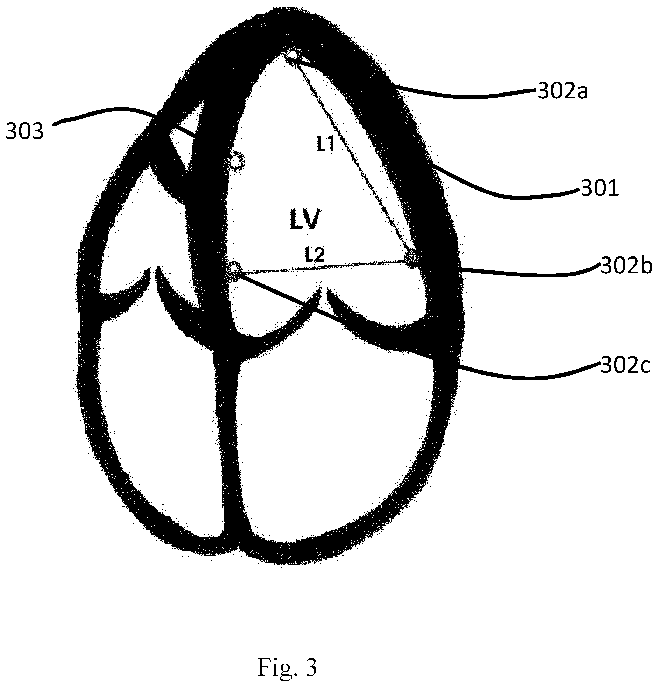

An example of an embodiment of the implanted part of the disclosure with one pressure sensor and two position sensors is shown in Fig. 3, with the left ventricle 301 being supplied with position sensors 302a - c and a pressure sensor 303.

The position sensors are tracked by an external means this being by use of ultrasound reflections from passive sensors to determine their relative displacement $\Delta x$ and absolute distance.

Further shown in Fig. 3, is an example of specific locations of the position sensors within the Left Ventricle. The figure shows the heart as seen in the Apical 4 Chamber view, one of the commonly used views during echocardiography. The distance between the position sensor deployed at the endocardial aspect of the apex of the ventricle and the position sensor deployed at the endocardial aspect of the lateral ventricular wall (L1) represents the longitudinal or axial dimension of the left ventricle which is commonly used by experts in the field to assess Global Longitudinal Shortening or Strain (GLS), while the distance between the two position sensors deployed at opposite locations on a cross-sectional plane of the ventricle (L2) represents the horizontal dimensions of the ventricle at a specified level.

Based on the assumptions that the left Ventricle resembles an ellipsoid shape, and that the distance L2 is similar to the distance perpendicular to it between the endocardial aspects of the ventricular wall at the same level, and taking simultaneous measurements of the 2 distances (L1 and L2) enables calculation of the Left Ventricular Volume according to the formula:

$$\text{LV Volume} = \frac{\pi}{6} \ x \ L1 \ x \ L2^2$$

Other formulae may, however, be used depending on the exact location in which the position sensors will be deployed as well as on data accumulated on specific patients as well as on patient groups and populations. For embodiments using passive ultrasound reflectors, the shape, size, design (solid or hollow), the surface features and material of the reflectors will have an effect on the nature and intensity of the reflections therefrom. The difference between the acoustic impedance of the reflector and that of the surrounding medium will determine the intensity of reflection or 'reflection coefficient'. The acoustic impedances of several materials are shown in the following table, along with the reflection coefficient expected at an interface between blood and the material in question; thus, the reflections expected from the materials in the table are all near 80% or above, and depending on their biocompatibility may be good candidates for reflectors. Non-biocompatible materials will be coated by or encapsulated within a biocompatible material. Such materials may include among others polyurethane, ceramic, medical grade silicon, titanium, Nitinol, etc.

Table 1 Reflection Coefficients for several materials, in blood

| Material | Acoustic Impedance [10^6 kg/m^2s] | Reflection coefficient |
|----------|-----------------------------------|------------------------|
| Air | 0.0004 | 0.999 |
| Alumina | 9.7 | 0.51 |
| Blood | 1.6 | 0 |
| Bone | 4.7 | .24 |
| Copper | 33.4 | .83 |
| Glass | 11.6 | .57 |
| Silver | 37.7 | .84 |
| Steel | 40.3 | .85 |
| Titanium | 27.6 | .79 |
| Tungsten | 97.8 | .93 |
| Zirconia | 42 | .86 |

It is within provision of the disclosure to use any material for the reflectors, formed into any shape; however, as the reflection will depend upon the orientation of the reflectors (which may change with heart wall movement, and cannot be controlled) it may be found useful to use regular polyhedral, cylinders, 'corner cubes', or spheres for the reflectors. The corner cube has the useful property that the reflected wave is entirely in the direction of the incoming wave. Whatever its form, the reflector may be hollow and preferably filled with air, to take advantage of further nearly-perfect reflections of the transmitted wave off of the interior walls of the reflector.

A smooth, hollow titanium or nitinol sphere of several wavelengths diameter is an example of a simple, biocompatible isotropic specular reflector. As medical ultrasound systems often use a 3.5MHz frequency with a wavelength of 0.44mm, this dictates use of a sphere of about 1mm diameter or larger. The size of the reflector will, however, be such that it would provide an optimal reflection for any wavelength being used currently or in the future for cardiac imaging.

By means of a calibration, the relation between the distance $\Delta x$ between the two (or more) sensors and the volume $V_{LV}$ of the left ventricle may be determined. Since the relationship between distance and volume is highly dependent on the position of the sensors within the ventricle and on the individual patient's heart size, morphology and function, a calibration will be obtained once during the procedure in which the sensors are deployed and then relied upon to determine volume $V_{LV}$ from the distance $\Delta x$. The calibration may be repeated when the patient undergoes cardiac catheterization for other indications. It further may be the case that the exact shape or coefficient of the calibration may prove to be unimportant, and rather that changes in the pressure-distance relationship over time are of sufficient clinical importance. In this case, the distance-pressure curve may be used as a proxy for the volume-pressure curve. It may also be the case that the calculated volume from 2 distances (as mentioned above for an ellipsoidal volume) may be used for the generation of pressure-volume loops without the need to calibrate with absolute volumetric measurements.

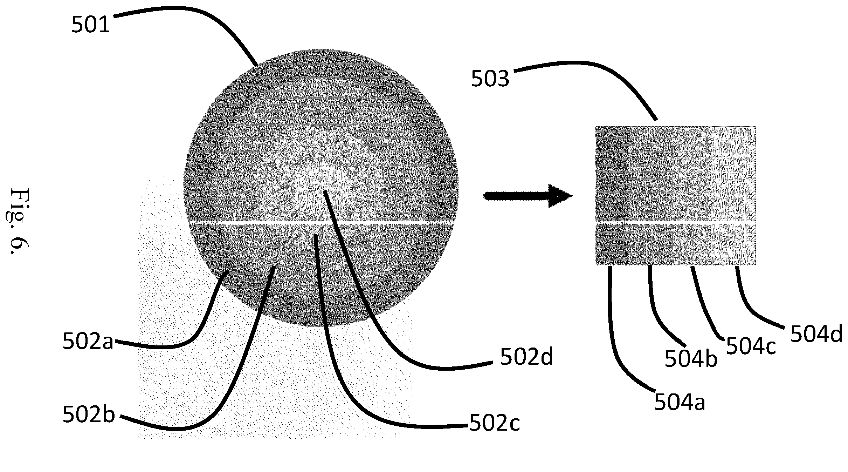

In another example, illustrated in Fig. 6 the location sensor (501) may include several layers (502a - d) each with a specific thickness and acoustic impedance. This will cause the reflected ultrasound beam to travel back from each interface between layers (502a - d) at a time relative to its thickness while the intensity of the signal will be proportional to the reflection coefficient at the interface between the layers (502a - d). Such a design will produce reflectors with specific "signatures" (503) allowing to use a multitude of reflectors (501) while enabling to distinguish between them as they are being tracked. The reflector-specific signature (503) can be presented as a "barcode" in which the width of each bar (504a - d) is relative to the thickness of the layer (502a - d) while the color or grayscale is relative to the acoustic impedance of each layer (502a - d). The bars (504a - d) can be arranged in an inside-out direction or vice versa. The barcode (503) presentation is not required for the practical implementation but may serve other uses of the system, e.g., the user may scan a barcode (503) of a certain reflector (501) and ask the system to locate only this specific reflector (501).

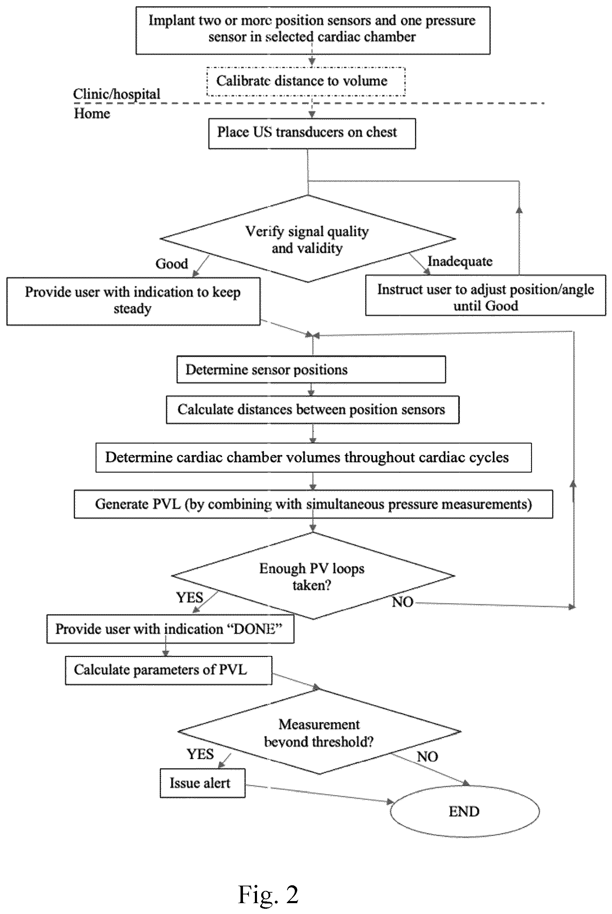

A simplified flowchart of one embodiment of the disclosure is shown in Fig. 2. First the sensors of the disclosure are implanted by means that will be described below. Once implanted, the calibration step described above is carried out, if necessary. These steps will generally be performed in a hospital setting. After being discharged, the patient, a family member or any other non-professional, can activate the external device to carry out a repeated sequence of distance and pressure measurements. This is done by moving the ultrasound transducer, or the belt holding an array of arrays of ultrasound emitting and receiving elements until receiving feedback from the external device in the form of a color indicator, sound or other, assuring a good signal quality has been achieved, and directing the patient to maintain this position as stable as possible. Then the device repeatedly measures the distances between position sensors, correlates them with pressure measurements until several pressure-volume loop (PVL) can be generated. When completed, the user receives another feedback "DONE" assuring a satisfactory examination session has been completed and the device may be switched off and removed from the chest. The PVL allows for several values to be calculated including measures of cardiac preload, afterload, myocardial contractility and relaxation. A step of measurement validity is carried out, and if the measurements are valid then the results are stored and may be sent to a network. The results are also compared to predefined thresholds, and if these are exceeded then an alert may be issued. The results may also be compared to the same patient's previous examinations such that deterioration or worsening are detected even if absolute values are still within the normal range. This may take the form of a local alarm as well as an alert sent to medical care providers, phone contacts, and so on. The frequency of measurements may be determined by the caring physician based on the type and severity of the patient's disease, and may range from one or a few measurements per day to one or a few measurements per week to allow for useful predictions of cardiac behavior to be made in time for interventions to be taken if necessary.

Although as described in Fig. 2, the invention is intended to be used at the outpatient or home setting, the high quality, operator-independent measurements provided by the technology and based on the disclosure, may be used in the hospital or clinic setting where the technology could be integrated into existing or future ultrasound devices and transducers. This will provide clinicians with a reliable, consistent way for assessing the existence and severity of heart failure, especially in the subset of patients suffering from diastolic dysfunction or HFpEF.

The position sensors are passive ultrasound reflectors.

The sensor can be made of any material adapted to reflect ultrasound energy.

This paragraph is not part of the invention.

In yet another embodiment, the sensors may be piezoelectric sonomicrometry crystals receiving power from an external source such as radiofrequency or ultrasound, emitting ultrasound signals to, and receiving ultrasound signals from other piezoelectric sonomicrometry crystals implanted in different locations within the cardiac chamber, and transmitting data to an external device by wireless means.

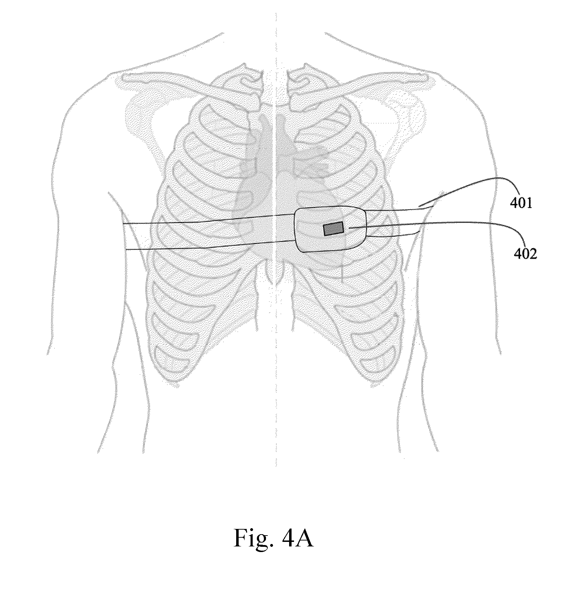

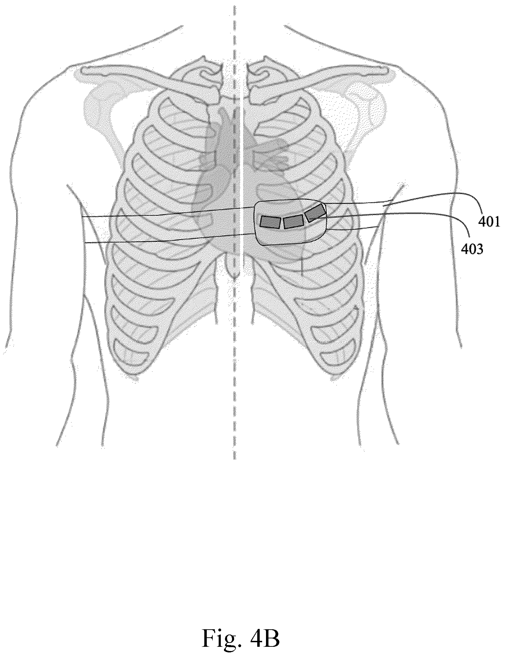

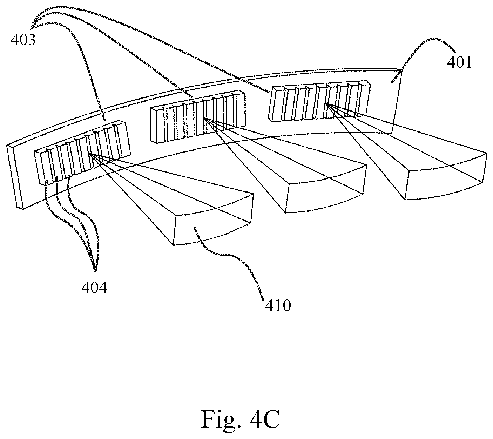

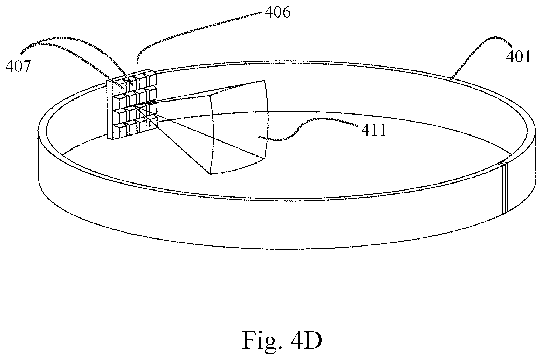

The ultrasound transducers are configured to act as a "Medical Radar" or data transmission system. To be able to determine the exact position of each sensor, the method requires a way of achieving true range multilateration or triangulation (similar to applications such as surveying, navigation, etc.). This can be achieved by either using a series of existing ultrasound transducers (i.e., a phased array) or by creating a series of custom-made arrays of ultrasound emitting and receiving elements angled relative to each other, either within a single probe or on a strip/belt etc., such that angulation is enabled. Such an implementation is shown in Fig. 4 where the phased arrays are placed on a band around the chest, where they may be positioned directly over the heart such that the signals are clear. An alternative embodiment of the method can be done by using a matrix ultrasound probe which scans at two planes perpendicular to each other and extracting spatial information from the transducer such that true range multilateration or triangulation may not be required.

An embodiment using a chest strap bearing an ultrasound probe is shown in Fig. 4A, where a belt or strap 401 is provided with a matrix ultrasound probe 402 that employs a set of transducer elements. Alternatively, a set of several linear (or phased) arrays 403 as shown in Fig. 4B may be used. The linear arrays will generally be angled relative to one another. Each array may be linear 403 (Fig. 4C) or itself of a matrix array form 406 (Fig. 4D). The arrays will generally have sets of transceiver elements (transmitting and receiving) 404, (Fig. 4C) although in some implementations separate elements may be used for transmitting and receiving. In Fig. 4D the transceiver elements 407 are shown in a square matrix. The relatively flat beam pattern 410 of the linear array, scanning a "slice" of an organ is shown in Fig. 4C, while the more volumetric (pyramidal) scanning pattern 411 of the matrix array is shown in Fig. 4D. The arrays and elements may be angled relative to each other. The belt bearing the array or arrays is worn obliquely on the chest over an intercostal space using an "acoustic window" between the ribs allowing the ultrasound signals to reach the heart without being obstructed by bone and cartilage. This approach is resembling the commonly used Apical 4 chamber view and the Parasternal Long Axis views.

It is within provision of the disclosure that suprasternal and substernal approaches may be applied using a hand-held transducer, without using such a belt. In the case of a matrix probe or matrix array as shown in Fig. 4D, since the probe scans in two planes perpendicular to each other, a single probe may suffice. It is within provision of the disclosure to use any of the available acoustic windows, including the aforementioned parasternal and apical windows, as well as the subcostal, suprasternal, and any other windows that may be found effective for practice of the invention.

It is within provision of the disclosure that various configurations of transducers or sets of transducers (including transceivers or separate transmitters and receivers) may be used to adapt to specific clinical needs.

The external unit may be divided into two physical parts, if for instance it is found that putting all necessary electronics, power supply, display means, communications means and so on upon a chest strap as in Fig. 4 is not possible. Thus, the chest strap of Fig. 4 may carry the ultrasound arrays, while a separate power/computing unit in communication with these arrays performs the data analysis, transmission to the cloud, supplies power, and so on. This separate power/computing unit may be in wired or wireless communication with the chest strap transducers.

It is within provision of the disclosure that the ultrasound elements include separate or unitized transmit and receive elements, and that the arrays be adapted for placement in contact with the skin, for example being arranged upon a flexible substrate adapted to conform to the body to promote good contact with the skin. To assure proper coupling of the ultrasound signal to the subject's skin, an encapsulated gel unit may be used between each transducer and the skin. Alternatively, a recess in the element holding the transducers opposite the skin may be filled by coupling gel prior to each examination.

The position and angle of the ultrasound transducers will be determined in a way which will ensure the acquisition of good quality signals from the passive reflectors depending on their specific locations within the heart. Such positions may include among others an intercostal space, substernal or suprasternal or others.

As will be clear to one skilled in the art, an advantage of phased arrays is the steerability of the main beam; this may be used for instance by scanning an area with the beam to simultaneously detect and track at least two reflectors, and then using the angle with the greatest response to achieve a high SNR. The number of elements in each transducer and their sequence of activation will be adapted to ensure optimal performance. Similarly, the delay in activation of elements will be optimized to minimize interference between their signals. When implemented by an array of arrays, the delay between activation of arrays can be modulated to achieve optimal performance and avoid signal interference.

Also, as known to one skilled in the art, in order to determine the location of a moving object using multiple ranges (distances) between the moving object and multiple spatially separated known locations. **In** an ultrasound probe, each transceiver element is an independent reference. The combination of several ultrasound probes, each consisting of multiple transceiver elements arranged in either a linear (or phased array) or a single matrix probe provides a plurality of measurements taken from different angles assuring a high accuracy and spatial resolution.

As mentioned, there is also provided a method and apparatus for determining the distance of two or more sensors from each other. The invention may also be applied to continuous or intermittent tracking of a plurality of sensors and determination of their relative position.

Specifically, the disclosure allows for measurement of the distance between two sensors or more within a cardiac chamber (i.e., the left ventricle). The position of each sensor relative to the external device is made by using true range multilateration or triangulation or alternatively the absolute positions of the sensors may be determined and then the distance calculated by simple geometric means.

When using a matrix ultrasound probe, the inherent properties of such a transducer, which scans a targeted volume in two planes perpendicular to each other, may enable the calculation of distances between sensors possible without traditional true range multilateration or triangulation methods.

Magnetic field means may also be used in addition to ultrasound means for relative or absolute position sensing of the wireless sensors. In this case the implanted sensors would have a specific magnetic structure adapted for remote detection.

The sensors can also be deployed in more than one cardiac chamber (i.e., left atrium and left ventricle, left and right ventricles, etc.) For some applications, the sensors can be deployed at locations not within cardiac chambers but adjacent to them (i.e., the coronary sinus, aorta, pulmonary arteries or veins, inferior or superior vena cava). This last sentence is not part of the invention which refers to "in or at" a cardiac chamber.

The sensors may be integrated into any existing or future implanted cardiac device such as artificial valves, PFO occluders, pacemakers, implantable cardioverter-defibrillator (ICD), left ventricular assist devices (LVAD), etc. The sensors are adapted with specific geometry, material, surface (smooth - rough) or echogenic coating, with an engineered response to specific energies/signals to create a specific reflection footprint ranging from "stealth" to noisy (these corresponding to non- or poorly-reflective, to highly-reflective, respectively).

The sensors (reflectors) may be in the form of an encapsulated cluster of microbubbles.

In one embodiment of the disclosure, the sensors may be brought to their target location by a trans-catheter approach, either via the arterial system to the left heart chambers, or the venous system to the right heart chambers. The left heart may also be reached by the venous route through a trans-septal puncture in the right atrium towards the left atrium.

It is expected that most sensor implantation procedures will be performed as an additional step during a cardiac catheterization or cardiac surgery (e.g., open, minimally invasive, trans-apical, transmural, etc.) performed for other purposes such as coronary angiography, valve repair or replacement, or the like. Alternatively, the sensor placement may also be a stand-alone procedure. Another method for deploying the sensors at their target location may employ an injection mechanism whereby the sensors are embedded in the myocardium and instantaneously covered by the surrounding tissue such that further fixation may not be required.

The sensors can be deployed at their target on the wall of a cardiac chamber by several methods including but not limited to attachment by a hook-like or spring like mechanism, self-expanding Nitinol anchors, or other means as will be clear to one skilled in the art. It is expected that the sensors will undergo endothelialization within a short period such that they will not be exposed to the blood in the heart thus eliminating the need for anticoagulation. The functionality of the passive reflectors will, however, not be affected by being covered by endothelium or embedded in the myocardium. Anchoring of the sensors to the myocardium is not only a safety feature. It assures that serial measurements over time are always made at the exact same location or plane which assures reliability and repeatability of measurements.

As discussed above, the sensors will be either made of, or covered by a biocompatible material. The material to be used has to be non-degradable to ensure lifetime endurance.

The measurements can be taken sporadically or in a continuous, real-time manner. Assuming a heart rate of 60-120bpm (cardiac cycle of 0.5-1.0 sec), a high enough sample frequency of will be used to achieve the desired temporal resolution, especially important not to miss the exact points of deflection of the PVL curve). Just as a non-limiting example, a sampling frequency in the range of 20 - 100 Hz will provide the desired temporal resolution.

In the preferred embodiment wherein passive ultrasound reflectors are implanted in the heart to determine volume, the external device will comprise an ultrasound transducer. For echocardiography, most transducers work at a frequency of 2-4 MHz allowing penetration to the required depth within the chest.

While the external device described in the disclosure may be custom made when applied for remote monitoring, the high quality, operator-independent measurements provided by the technology and based on the disclosure, may be used in the hospital or clinic setting where the technology could be integrated into existing or future ultrasound devices and transducers. This will provide clinicians with a reliable, consistent way for assessing the existence and severity of heart failure, especially in the subset of patients suffering from diastolic dysfunction or HFpEF.

It is within provision of the disclosure that the external device scans a certain "slice" of the organ when using a linear (or phased) array, while a matrix arrangement of piezoelectric elements allows their phasic firing to produce an ultrasound beam that can be steered in vertical (axial), lateral (azimuthal) and antero-posterior (elevation) directions in order to acquire a volumetric (pyramidal) data set. These scanning properties assure that at least two sensors are identified within that slice or volume and tracked for at least a few cardiac cycles at each session.

It is within provision of the disclosure that the external device provides the user with feedback assuring that the signal reflected from at least two sensors is of sufficient quality to enable reliable results. This may be accomplished by various self-check means such as thresholding on values of pressure and volume, the area of the PVL, time derivatives of volume and/or pressure, signal strength, signal-to-noise ratio, and so on.

Unlike other imaging modalities, there is no need to acquire images for producing the desired results. Images may, however, be displayed as a side product of the system as part of the feedback provided to the user, such as highlighting the identified location sensors within the overall ultrasonographic scan.

The external device performs certain calculations on the measurements, including deriving cardiac chamber volume from one or more distance measurements, and calculating the pressure-volume loops as they accumulate over time, and functions of this loop such as End-Systolic pressure Volume Relationship (ESPVR), End-Systolic Elastance (EEs) - the slope of ESPVR considered to be the best indicator of myocardial contractility, End-Diastolic Pressure Volume Relationship (EDPVR) Arterial Elastance (Ea) - a measure of afterload, and the like.

The results calculated by the external device may be stored, transmitted and further analyzed by cellular phones, cloud, other computers, etc.

As mentioned, the measurements of the device can be correlated with measurements of other parameters such as pressure within one or more cardiac chambers. The pressure sensing may be accomplished by means of separate wireless sensors, or alternatively integrated with the volume sensing means.

In some embodiments, one of the location sensors may be integrated with a pressure sensor (for example being encapsulated together) such that the total number of sensors can be reduced.

As an example of the utility of the invention, left ventricular volume measured throughout cardiac cycles when correlated with continuous pressure measurements can yield pressure-volume loops indicative of myocardial function.

It is further within provision of the disclosure that information be derived regarding function or dysfunction of cardiac valves on the left side of the heart (i.e., mitral or aortic stenosis or regurgitation). The same may be applied to valves in the right side of the heart (i.e., Tricuspid or Pulmonic stenosis or regurgitation).

The measurements may also be correlated with or gated with the electrical activity of the heart (ECG).

The system may also integrate measurements derived from one or more miniaturized three-axis (3D) accelerometers. When positioned in the atria, an accelerometer can provide information on dysrhythmias (i.e., atrial fibrillation) which would enable refining of PV loops, while when positioned in a cardiac ventricle, identification of dyskinetic regions can improve the assessment of the displacement between the position sensors and the orientation thereof.

Measurements may be modulated by external manipulations (brachial or femoral cuffs to increase afterload, Valsalva maneuver or positional change to reduce preload) in order to produce a series of pressure-volume loops displaced relative to each other thus enabling the calculation of end-systolic pressure-volume relationship (ESPVR), end-diastolic pressure-volume relationship (EDPVR) and other parameters of myocardial function.

The disclosure is primarily intended for continuous or intermittent monitoring with emphasis of outpatient environment of patients suffering from congestive heart failure (CHF) but also valvular heart disease, intracardiac shunts, etc. However, the high quality, operator-independent measurements provided by the technology and based on the disclosure, may be used in the hospital or clinic setting where the technology could be integrated into existing or future ultrasound devices and transducers. This will provide clinicians with a reliable, consistent way for assessing the existence and severity of heart failure, especially in the subset of patients suffering from diastolic dysfunction or HFpEF.

This paragraph is not part of the invention.

It is within provision of the disclosure that it be applied to any organ in the human body. As an example of further uses, the disclosure can be used to continuously monitor the excursion of movement of the diaphragm between the chest and the abdominal cavity, an indicator of pulmonary function and other parameters in chronic lung disease, some neurological conditions, and in the critically ill. Position sensors are attached or implanted accordingly to provide the necessary data, as the skilled person will realize.

The ultrasound transducers to be used in the disclosure may include wireless handheld transducers, fingertip probes, wearable transducers such as a "beltlike" set of ultrasound transducers (set of arrays), a combination of arrays of transmitters angled relative to each other, ultrasound on a chip, with capacitive micromachined US transducers as an alternative to piezoelectric transducers, or externally powered wireless implanted sonomicrometry crystals. Although the invention is primarily intended to be used by patients at home (after placement of the implanted elements of the disclosure) it may provide significant value also at hospital and Point of Care (POC) clinics. The current practice of echocardiography is often subject to interobserver variability in determining critical parameters such as Ejection Fraction (EF), due to measurement variability such as measurements by different operators being performed in slightly different planes. This issue is solved by examples of the invention, as it relies on detection of the implanted sensors which always stay at the same location.

Another advantage of examples of the invention over imaging ultrasound is that it does not rely on acquisition and analysis (by expert or AI) of high-resolution ultrasound images, a method which will have unavoidable inaccuracies due (for instance) to operator variability and/or inaccuracies due to estimation of a 3D volume from a 2D image. The passive ultrasound reflectors of examples of the invention will generate a high SNR, and will thus be detectable with a high level of reliability and consistency.

The ability to generate and analyze pressure-volume loops of a cardiac chamber as described in the disclosure provides a reliable means for the monitoring of a large group of patients suffering from heart failure with a preserved ejection fraction (HFpEF) where measurements of volumes alone may not provide the clinically relevant information and proper assessment of the severity of the disease.

The foregoing description and illustrations of the embodiments and examples of the invention or disclosure has been presented for the purposes of illustration. It is not intended to be exhaustive.

Any term that has been defined above and used in the claims, should be interpreted according to this definition.

The reference numbers in the claims are not a part of the claims, but rather used for facilitating the reading thereof. These reference numbers should not be interpreted as limiting the claims in any form.

## Claims

**1.** An external device for wireless cardiac diagnostics, said external device comprises:

ultrasound transducers (404) adapted to be arranged on a chest of a subject or in a hand-held device; and
a processor unit configured to:

i. provide an ultrasound signal from said ultrasound transducers and obtain related reflector signals, based on said reflector signals determine displacement data related to displacements of a set of wireless position sensors (302a-c) relative to each other, wherein said wireless position sensors are deployed in or at a cardiac chamber, wherein said wireless position sensors are passive ultrasound reflectors having high ultrasound reflectivity;

ii. obtain pressure data from a pressure sensing means (303) adapted for sensing the pressure within said cardiac chamber;

iii. determine a pressure-volume loop of a pressure and volume of said cardiac chamber based on said displacement data and pressure data;

iv. and determine cardiac health based on said pressure-volume loop;

whereby said device is configured to wirelessly, non-invasively, and continuously determine said cardiac health.

2. A system for wireless cardiac diagnostics comprising:

a. a set of wireless position sensors (302a - c) configured to be deployed in a cardiac chamber, wherein said wireless position sensors are passive ultrasound reflectors having high ultrasound reflectivity;

b. pressure sensing means (303) adapted for sensing the pressure within said cardiac chamber;

c. an external device according to claim 1; and

whereby said system is configured to wirelessly, non-invasively, and continuously determine said cardiac health.

3. The system of claim 2, wherein said wireless position sensors are solid or hollow forms selected from the group consisting of: sphere, coil, cylinder, polyhedron, corner cube, ellipsoid, ring.

4. The system of claim 2, wherein said external device is configured to use true range multilateration or triangulation to determine said displacement of said wireless position sensors.

5. The system of claim 2, wherein said external device is configured to extract and process raw spatial data from ultrasound transducer(s) to determine said displacement of said wireless position sensors.

6. The system of claim 2, wherein said pressure sensing means is wireless.

7. The system of claim 2, wherein said pressure sensing means is incorporated into one or more of said wireless position sensors.

8. The system of claim 2, wherein said position sensors comprises of several layers each with a specific thickness and acoustic impedance.

9. The system of claim 8, wherein said position sensors are reflectors each with a specific signature pattern.

10. A software for cardiac health determination, including executable instructions for a processing unit of an external device according to claim 1 and performing a method of cardiac health determination including noninvasive determination of cardiac pressure-volume loops, said method:

a. continuously obtaining reflectance signal data from an ultrasound transducer (404) wherein said reflectance signal data relates to passive ultrasound reflectors (302a-c) having high ultrasound reflectivity; determining the displacement or displacements between two or more position sensors (302a-c) previously affixed in or at a cardiac chamber, thus providing displacement data;

b. continuously obtaining pressure data and determining the pressure in said cardiac volume, wherein said pressure data is obtained from a pressure sensor (303) previously implanted in said cardiac chamber or a non-invasive cardiac pressure sensor or method, thus providing pressure data;

c. forming at least one pressure-volume loop from said displacement and pressure measurements' data;

d. calculating parameters of said at least one pressure-volume loop adapted to indicate cardiac health;

whereby cardiac health is determined wirelessly, non-invasively, continuously.

11. The software of claim 10, wherein said external device uses true range multilateration or triangulation to determine said displacement of said wireless position sensors.

12. The software of claim 10, wherein said pressure sensing means is incorporated into one or more of said wireless position sensors.

**Patentansprüche**

1. Ein externes Gerät für die drahtlose Herzdiagnostik, wobei das externe Gerät umfasst:

   Ultraschallwandler (404), die so ausgelegt sind, dass sie auf der Brust einer Person oder in einem Handgerät angeordnet werden können; und
   eine Prozessoreinheit, die so konfiguriert ist, dass sie:

   i. ein Ultraschallsignal von besagten Ultraschallwandlern bereitstellt und zugehörige Reflektorsignale erfasst und auf der Grundlage der besagten Reflektorsignale Verschiebungsdaten in Bezug auf Verschiebungen einer Reihe von drahtlosen Positionssensoren (302a-c) relativ zueinander bestimmt, wobei besagte drahtlose Positionssensoren in oder an einer Herzkammer angeordnet sind, wobei besagte drahtlose Positionssensoren passive Ultraschallreflektoren mit hoher Ultraschallreflektivität sind;
   ii. Druckdaten von einer Druckerfassungseinrichtung (303) erfasst, die so ausgelegt ist, dass sie den Druck innerhalb besagter Herzkammer erfasst;
   iii. eine Druck-Volumen-Kurve eines Drucks und Volumens besagter Herzkammer auf der Grundlage besagter Verschiebungsdaten und Druckdaten bestimmt;
   iv. und die Herzgesundheit auf der Grundlage besagter Druck-Volumen-Kurve bestimmt;

   wobei besagte Vorrichtung so konfiguriert ist, drahtlos, nicht-invasiv und kontinuierlich besagte Herzgesundheit zu bestimmen.

2. Ein System zur drahtlosen Herzdiagnostik, umfassend:

   a. einen Satz drahtloser Positionssensoren (302a-c) die so konfiguriert sind, dass sie in einer Herzkammer eingesetzt werden, wobei besagte drahtlose Positionssensoren passive Ultraschallreflektoren mit hoher Ultraschallreflektivität sind;
   b. Druckmessmittel (303) die zum Erfassen des Drucks innerhalb besagter Herzkammer ausgelegt sind;
   c. ein externes Gerät gemäß Anspruch 1; und
   wobei besagtes System so konfiguriert ist, dass es besagte Herzgesundheit drahtlos, nicht-invasiv und kontinuierlich bestimmt.

3. Das System nach Anspruch 2, wobei besagte drahtlose Positionssensoren feste oder Hohlkörper sind, ausgewählt aus der Gruppe bestehend aus: Kugel, Spule, Zylinder, Polyeder, Eckwürfel, Ellipsoid, Ring.

4. Das System nach Anspruch 2, wobei besagte externe Vorrichtung so konfiguriert ist, dass sie True-Range-Multilateration oder Triangulation verwendet, um besagte Verschiebung besagter drahtloser Positionssensoren zu bestimmen.

5. Das System nach Anspruch 2, wobei besagtes externes Gerät so konfiguriert ist, dass es Rohraumdaten aus Ultraschallwandlern extrahiert und verarbeitet, um die Verschiebung besagter drahtloser Positionssensoren zu bestimmen.

6. Das System nach Anspruch 2, wobei besagte Drucksensoreinrichtung drahtlos ist.

7. Das System nach Anspruch 2, wobei besagte Drucksensoreinrichtung in einen oder mehrere der besagten drahtlosen Positionssensoren integriert ist.

8. Das System nach Anspruch 2, wobei besagte Positionssensoren aus mehreren Schichten mit jeweils einer bestimmten Dicke und akustischen Impedanz bestehen.

9. Das System nach Anspruch 8, wobei besagte Positionssensoren Reflektoren mit jeweils einem spezifischen Signaturmuster sind.

10. Eine Software zur Bestimmung der Herzgesundheit, einschließlich ausführbarer Anweisungen für eine Verarbeitungseinheit eines externen Geräts gemäß Anspruch 1 und zur Durchführung eines Verfahrens zur Bestimmung der Herzgesundheit, einschließlich der nicht-invasiven Bestimmung von Herz-Druck-Volumen-Kurven, wobei besagtes Verfahren Folgendes umfasst:

  a. das kontinuierliche Erfassen von Reflexionssignaldaten von einem Ultraschallwandler (404), wobei sich besagte Reflexionssignaldaten auf passive Ultraschallreflektoren (302a-c) mit hoher Ultraschallreflektivität beziehen; das Bestimmen der Verschiebung oder Verschiebungen zwischen zwei oder mehr Positionssensoren (302a-c), die zuvor in oder an einer Herzkammer befestigt wurden;
  b. das kontinuierliche Erfassen von Druckdaten und das Bestimmen des Drucks in besagtem Herzvolumen, wobei besagte Druckdaten von einem Drucksensor (303) erfasst werden, der zuvor in die Herzkammer implantiert wurde, oder einem nicht-invasiven Herzdrucksensor invasiven Herzdrucksensor oder Verfahren, wodurch Druckdaten bereitgestellt werden;
  c. das Bilden mindestens einer Druck-Volumen-Kurve aus besagten Verschiebungs- und Druckmessdaten;
  d. das Berechnen von Parametern der mindestens einen Druck-Volumen-Kurve, die dazu geeignet sind, die Herzgesundheit anzuzeigen;
  wobei die Herzgesundheit drahtlos, nicht-invasiv und kontinuierlich bestimmt wird.

11. Die Software nach Anspruch 10, wobei besagtes externes Gerät True-Range-Multilateration oder Triangulation verwendet, um die Verschiebung besagter drahtloser Positionssensoren zu bestimmen.

12. Die Software nach Anspruch 10, wobei besagte Drucksensormittel in einen oder mehrere der besagten drahtlosen Positionssensoren integriert sind.

**Revendications**

1. Dispositif externe destiné à des diagnostics cardiaques sans fil, ledit dispositif externe comprenant :

  des transducteurs ultrasonores (404) conçus pour être agencés sur une poitrine d'un sujet ou dans un dispositif tenu à la main ; et
  une unité de processeur configurée pour :

    i. fournir un signal ultrasonore à partir desdits transducteurs ultrasonores et obtenir des signaux de réflecteur associés, sur la base desdits signaux de réflecteur déterminer des données de déplacement associées à des déplacements d'un ensemble de capteurs de position sans fil (302a-c) les uns par rapport aux autres, où lesdits capteurs de position sans fil sont déployés dans ou au niveau d'une cavité cardiaque, où lesdits capteurs de positon sans fil sont des réflecteurs d'ultrasons passifs présentant une réflectivité d'ultrasons élevée ;
    ii. obtenir des données de pression à partir d'un moyen de détection de pression (303) conçu pour détecter la pression à l'intérieur de ladite cavité cardiaque ;
    iii. déterminer une boucle pression-volume d'une pression et d'un volume de ladite cavité cardiaque sur la base desdites données de déplacement et données de pression ;
    iv. et déterminer la santé cardiaque sur la base de ladite boucle pression-volume ;

  grâce à quoi ledit dispositif est configuré pour déterminer de manière sans fil, non invasive et continue ladite santé cardiaque.

2. Système destiné à des diagnostics cardiaques sans fil comprenant :

  a. un ensemble de capteurs de position sans fil (302a-c) configurés pour être déployés dans une cavité cardiaque, où lesdits capteurs de positon sans fil sont des réflecteurs d'ultrasons passifs présentant une réflectivité d'ultrasons élevée ;
  b. un moyen de détection de pression (303) conçu pour détecter la pression à l'intérieur de ladite cavité cardiaque ;
  c. un dispositif externe selon la revendication 1 ; et

grâce à quoi ledit système est configuré pour déterminer de manière sans fil, non invasive et continue ladite santé cardiaque.

3. Système selon la revendication 2, dans lequel lesdits capteurs de position sans fil sont des formes creuses ou pleines sélectionnées parmi le groupe constitué : d'une sphère, d'une hélice, d'un cylindre, d'un polyèdre, d'un cube d'angle, d'un ellipsoïde, d'un cercle.

4. Système selon la revendication 2, dans lequel ledit dispositif externe est configuré pour utiliser une multilatération ou une triangulation de plage vraie pour déterminer ledit déplacement desdits capteurs de position sans fil.

5. Système selon la revendication 2, dans lequel ledit dispositif externe est configuré pour extraire et traiter des données brutes provenant d'un ou plusieurs transducteurs ultrasonores pour déterminer ledit déplacement desdits capteurs de position sans fil.

6. Système selon la revendication 2, dans lequel ledit moyen de détection de pression est sans fil.

7. Système selon la revendication 2, dans lequel ledit moyen de détection de pression est incorporé dans un ou plusieurs desdits capteurs de position sans fil.

8. Système selon la revendication 2, dans lequel lesdits capteurs de position sont constitués de plusieurs couches ayant chacune une épaisseur et une impédance acoustique spécifiques.

9. Système selon la revendication 8, dans lequel lesdits capteurs de position sont des réflecteurs ayant chacun une signature spécifique.

10. Logiciel destiné à la détermination de santé cardiaque, incluant des instructions exécutables pour une unité de traitement d'un dispositif externe selon la revendication 1 et réalisant un procédé de détermination de santé cardiaque incluant une détermination non invasive de boucles de pression-volume cardiaques, ledit procédé :

   a. obtenant en continu des données de signal de réflectance à partir d'un transducteur ultrasonore (404) où lesdites données de signal de réflectance se rapportent à des réflecteurs d'ultrasons passifs (302a-c) présentant une réflectivité d'ultrasons élevée ; déterminant le déplacement ou les déplacements entre deux capteurs de position (302a-c) ou plus préalablement fixés dans ou au niveau d'une cavité cardiaque, fournissant ainsi des données de déplacement ;
   b. obtenant en continu des données de pression et déterminant la pression dans ledit volume cardiaque, où lesdites données de pression sont obtenues à partir d'un capteur de pression (303) préalablement implanté dans ladite cavité cardiaque ou d'un capteur ou procédé de pression cardiaque non invasif, fournissant ainsi des données de pression ;
   c. constituant au moins une boucle pression-volume à partir desdites données de déplacement et de mesure de pression ;
   d. calculant des paramètres de ladite au moins une boucle pression-volume conçus pour indiquer la santé cardiaque ;

   grâce à quoi la santé cardiaque est déterminée de manière sans fil, non invasive et continue.

11. Logiciel selon la revendication 10, où ledit dispositif externe utilise une multilatération ou une triangulation de plage vraie pour déterminer ledit déplacement desdits capteurs de position sans fil.

12. Logiciel selon la revendication 10, où ledit moyen de détection de pression est incorporé dans un ou plusieurs desdits capteurs de position sans fil.

| Signal source and receiving module(s) |
| --- |

| Processing module |
| --- |

| Relative position determination means |
| --- |

| Display means |
| --- |

| Battery/ power supply |
| --- |

| Communications module |
| --- |

| Position Sensor 1 |
| --- |

| Position Sensor 2 |
| --- |

| Position Sensor N |
| --- |

| Pressure Sensor |
| --- |

Fig. 1

Fig. 2

Fig. 3

Fig. 4A

401
403

Fig. 4B

Fig. 4C

Fig. 4D

Fig. 5. (Prior Art)

Fig. 6.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10078893 B
- US 2022265157 A1, Charthad Jayant
- US 20220825 A1
- US 2018177486 A1, Gifford, III, Hanson
- US 20180628 A1
- US 6498944 B
- US 10918858 B

**Non-patent literature cited in the description**

- **SAGAWA**. The End-systolic Pressure-Volume Relation of the Ventricle: Definition, Modifications, and Clinical Use. *Circulation*, 1981, vol. 63 (6), 1223-1227
- **BAAN et al.** Continuous measurement of left ventricular volume in animals and humans by conductance catheter. *Circulation*, 1984, vol. 70 (5), 812-823
- **BURKHOFF**. Pressure-Volume Loops In Clinical Research. *J. American College of Cardiology*, 2013, vol. 62 (13), 1173-1176
- **GUTERMAN**. *Automatic Left Ventricular Function Evaluation*